# EUROPEAN PATENT APPLICATION

(11) **EP 1 362 609 A1**
(43) Date of publication of application: **19.11.2003**
(21) Application number: 02425309.8
(22) Date of filing: 16.05.2002
(51) Int. Cl.: A61M 5/50, A61M 5/32

(54) **Disposable safety syringe with automatically guided outer sleeve**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A disposable syringe (100) comprises a syringe body (1) hollow on the inside, a plunger (4) sliding inside the syringe body (1) and provided at the rear with a stem (41) that can be operated manually, an injection needle that can be attached to the front end (13) of the syringe body (1), by means of a needle-carrier (3), a sleeve (5) mounted slidably over the syringe body (1) to pass from a retracted position of use of the syringe to a forward position of safety in which it covers the needle (2), spring means (7) disposed under compression between the sleeve (5) and the syringe body (1), locking means (56, 58, 12) provided in the rear part of the sleeve (5) and of the syringe body (1) in mutual engagement to lock the sleeve in a retracted position of use, operating means (44) disposed in the stem (41) to disengage the locking means when the plunger (4) reaches the end of the injection stroke, and guide means (60) disposed in the sleeve and cooperating with complementary guide means (33) integral with the syringe body (1) to guide the axial movement of the sleeve (5) with respect to the syringe body.

## Description

The present invention refers to a disposable safety syringe provided with an outer sleeve that is automatically guided into a safety position once the injection has been completed.

As is known, a syringe generally comprises a cylindrical body open at the rear to receive a plunger. A needle hollow on the inside is mounted at the head of the syringe. The liquid contained in a vial is drawn into the syringe body through the needle upon retraction of the plunger. The liquid contained in the syringe body is injected through the needle into the patient's body by pressing on the plunger.

Because of hygiene rules and to avoid transmission of infectious diseases, syringes must generally be used only once and then discarded. For this reason, there is a growing demand on the market for disposable syringes that are able to prevent further use thereof.

Moreover, syringes generally present drawbacks from the point of view of safety. In fact, once the syringe has been used, the needle remains exposed at the fore end of the syringe, with the risk of accidental injury or needle sticks.

Patent application PCT WO 99/37345 discloses a disposable safety syringe which provides a needle-covering sleeve mounted axially on the syringe body and sliding from a retracted position, in which it leaves the needle exposed to allow injection, to a forward or extended position in which it completely covers the needle, preventing reuse of the syringe and acting as a guard against accidental needle sticks.

Once the injection has been carried out, the sleeve is automatically brought into the extended safety position, by means of an automatic mechanism and without any intervention by the user. However, this solution holds a certain complexity due to the presence of various additional elements for operation of the automatic mechanism.

Furthermore, the telescopic movement of the sleeve on the syringe body is not guided, with the result of possible jamming and dysfunctions of the safety device of the syringe.

The object of the present invention is to eliminate the drawbacks of the prior art, providing a disposable safety syringe that is practical, versatile, economical and easy to make.

Another object of the present invention is to provide such a disposable syringe that is able to prevent further attempts at use.

Yet another object of the present invention is to provide such a disposable syringe that is extremely safe and able to prevent accidental injury after use thereof.

These objects are achieved in accordance with the invention with the characteristics listed in appended independent claim 1.

Advantageous embodiments of the invention are apparent from the dependent claims.

The disposable syringe according to the invention comprises a syringe body hollow on the inside and open at the front and rear, a plunger sliding inside the syringe body with an injection stroke extending from a retracted syringe filling position to a forward syringe emptying position, and an injection needle that can be attached to the fore end of the syringe body, by means of a needle-carrier. The plunger is provided at the rear with a stem that can be operated manually and brought out or extended out of the syringe body through the rear end thereof.

The syringe further comprises a sleeve slidably mounted over the syringe body, to pass from a retracted use position for use of the syringe to a forward safety position, in which it covers the needle. Spring means are disposed under compression between the sleeve and the syringe body to urge the axial movement of the sleeve into the forward safety position with respect to the syringe body.

The sleeve is blocked in the use position by means of locking means, in reciprocal engagement, provided in the rear part of the sleeve and of the syringe body. In the rear part of the stem, on the other hand, operating means are provided to disengage said locking means when the plunger reaches the end of the injection stroke, so as to allow the action of the spring means that automatically generate the axial movement of the sleeve with respect to the syringe body.

The peculiarity of the disposable syringe according to the invention is represented by the fact that said sleeve comprises guide means cooperating with complementary guide means integral with the syringe body to guide the axial movement of the sleeve with respect to the syringe body.

The advantages of the disposable syringe according to the invention are evident. In fact, once the injection has been completed, the sleeve is automatically guided in its telescopic movement on the syringe body, so as to cover the needle. In this manner the needle is in a safety position and thus accidental needle sticks and any attempts at reusing the syringe are avoided.

Further characteristics of the invention will be made clearer by the detailed description that follows, referring to purely exemplary and therefore non-limiting embodiments thereof, illustrated in the appended drawings, in which:
Figure 1 is an exploded axonometric view illustrating the disposable safety syringe according to the invention;
Figure 2 is a view from the rear of the needle-carrier of the syringe of Figure 1;
Figure 3 is an axial sectional view of the needle-carrier, taken along the sectional plane III-III of Figure 2;
Figure 4 is an axial sectional view of the sleeve of the safety syringe according to the invention;
Figure 5 is a side view of the syringe of Figure 1 assembled and in position ready for use;
Figure 6 is an axial sectional view of the assembled syringe, ready for use, taken along the sectional plane VI-VI of Figure 5;
Figure 7 is an axial sectional view, like Figure 6, of the syringe according to the invention, when the plunger has reached the end of the injection stroke;
Figure 8 is a side view of the syringe according to the invention, in the safety position;
Figure 9 is an axial sectional view of the syringe according to the invention in the safety position, taken along the sectional plane IX-IX of Figure 8, in which the syringe body has not been sectioned.

A disposable safety syringe according to the invention, designated as a whole by reference numeral 100, is described with the aid of the figures.

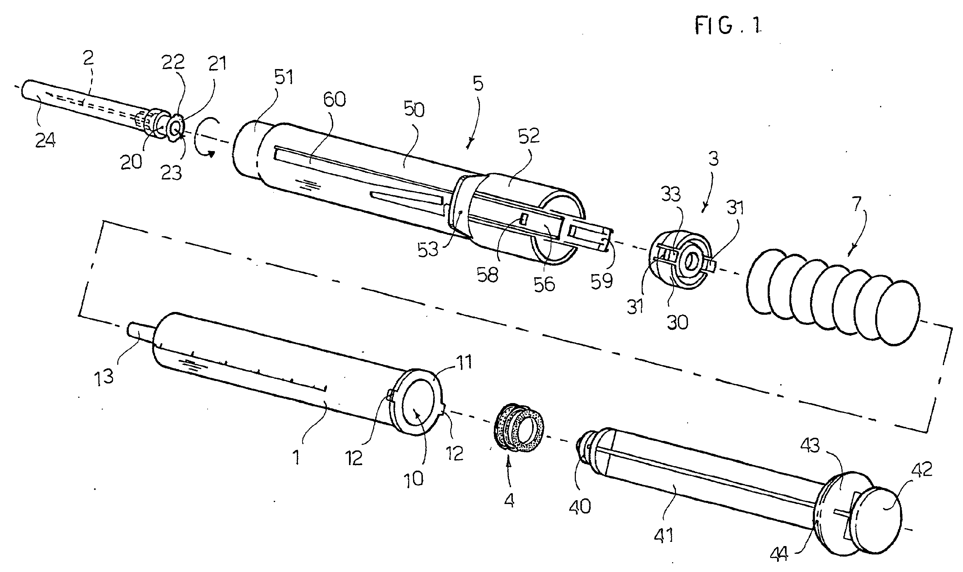

With reference for now to Figure 1 in particular, the syringe 100 comprises a cylindrical body 1, hollow on the inside, defining a cylindrical chamber 10. The rear end of the body 1 is outwardly open and has an annular collar 11 which protrudes radially outward. Two tongues or nibs 12 which protrude radially outward in diametrically opposite positions are provided on the collar 11.

The fore end of the body 1 ends in a head 13, open toward the outside, with a substantially frusto-conical shape and a smaller diameter than the body 1.

A needle 2 is supported by a cylindrical or frusto-conical support 20, hollow on the inside, having an axial chamber 23 able to receive the head 13 of the syringe body. The support 20 of the needle has at its free end a collar 21 with two tongues 22 protruding radially in diametrically opposite positions. The tongues 22 can be replaced by a thread. A needle cover hood 24 engages with the support 20 to cover the needle 2.

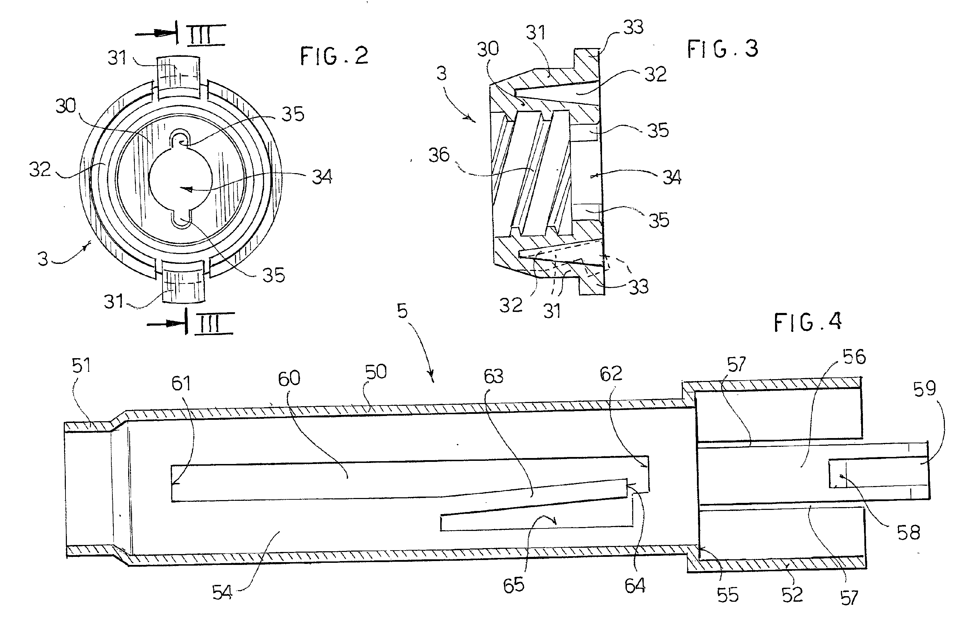

A needle-carrier device 3, also shown in Figures 2 and 3, comprises a cylindrical body 30 that has two flexible tongues 31 disposed in diametrically opposite directions. The flexible tongues 31 are formed by means of notches 32, so as to be able to bend along the notches 32 moving away from or toward the body 30 of the needle-carrier. The flexible tongues 32 end with teeth or nibs 33 protruding radially outward.

The needle-carrier device 3 has an axial through channel 34 ending in an internal thread 36. The entrance of the channel 34 has two lateral guiding apertures 35, able to allow the passage of the tongues 22 of the needle support 20, which screw into the inner thread 36 of the needle-carrier device 3.

A plunger 4 can slide with a tight seal inside the chamber 10 of the syringe body 1. The plunger 4 is mounted on the head 40 of a stem 41 having a cross-shaped cross section. The stem 41 ends at the rear with a disc-shaped flange 42 that provides an abutment surface for the user's finger during the injection.

Near the rear flange 42, around the stem 41, an operating crown 43 that has a tapered front side surface 44 is provided. The operating crown 43 can be made in a single body with the rear flange 42 of the stem 41.

As shown also in Figure 4, a safety device of the syringe according to the invention is designated by reference numeral 5 and takes the form of a sleeve with a substantially cylindrical, internally hollow body 50, having an axial chamber 54 open at the front and rear. The sleeve 5 has a front part 51 with a smaller diameter and a rear part 52 with a larger diameter with respect to the central part 50 of the sleeve body. The inside diameter of the central part 50 of the sleeve body is slightly larger than the outside diameter of the syringe body 1, so that the sleeve 5 can slide axially on the syringe body.

Two stiff tongues 53 which protrude radially outward are provided between the central part 50 and the rear part 52 of the sleeve body, to form a resting surface for the user's fingers. Again between the central part 50 and the rear part 52 of the sleeve body, an annular abutment surface 55 is formed which protrudes radially inward.

Two longitudinal flexible tongues 56 disposed in diametrically opposite positions are provided in the rear part 52 of the sleeve body. Each tongue 56 is defined by two longitudinal parallel notches 57 on the rear part 52 of the sleeve body, so as to be able to bend outward with respect to the sleeve body.

Each longitudinal tongue 56 has a through slot 58 disposed in a substantially central position, able to receive the radial tongues 12 of the collar of the syringe body 1. Moreover each tongue 56 protrudes rearward with respect to the rear edge of the rear part 52 of the sleeve and has in its rear end, a substantially tapered inner surface 59, able to cooperate with the tapered surface 44 of the operating crown 43 of the stem 41.

Two longitudinal slots 60 disposed in diametrically opposite positions, able to receive the teeth 33 of the flexible tongues 31 of the needle-carrier device 3 are formed in the central part of the sleeve body. Each longitudinal slot has a front abutment and end-of-stroke surface 61 and a rear abutment and end-of-stroke surface 62.

In the rear part of the longitudinal slot 60, there extends a flexible tongue 63 which has an abutment surface 64 disposed opposite to the rear abutment surface 62 of the longitudinal slot 60. Each flexible tongue 63 is defined by a longitudinal notch 65 made in the central part 50 of the sleeve body, parallel to the longitudinal slot 60. In this manner the elastic tongue 63, which normally occupies part of the longitudinal slot 60 forming a narrowing in said slot, can bend in the notch 65, eliminating the narrowing of said slot.

Lastly, the syringe 100 comprises a spiral spring 7, destined to be housed in the rear part 52 of the body of the sleeve and able to allow the passage of the syringe body 1 therein.

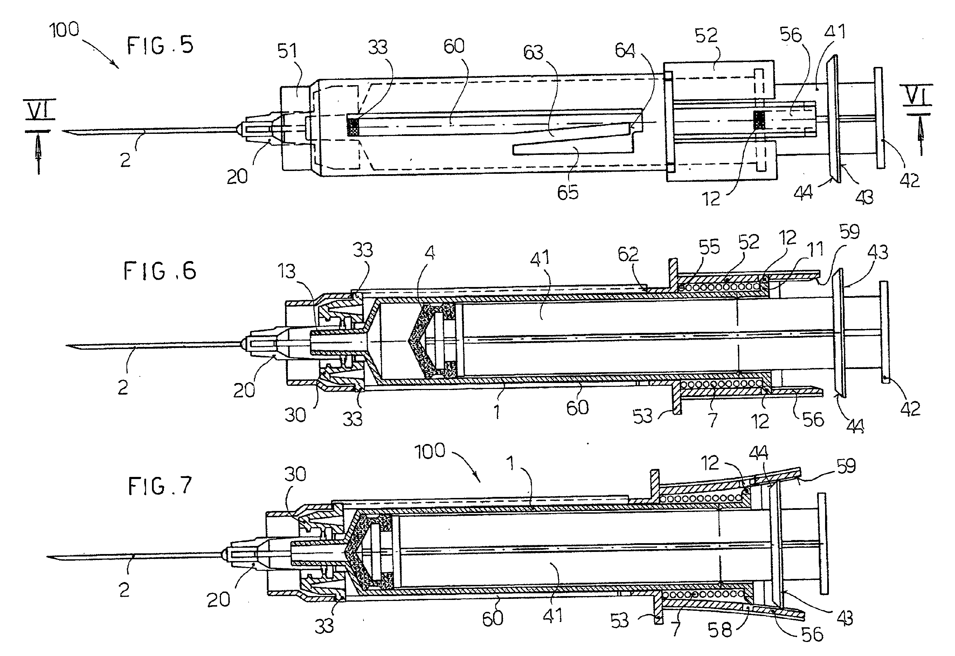

Assembly of the syringe 100 according to the invention will now be described, purely by way of example, also with the aid of Figures 5 and 6.

The plunger 4 is mounted at the head 40 of the stem 41 and inserted inside the syringe body 1. The spring 7 is inserted from the front part on the syringe body 1, until one end of the spring 7 abuts against the collar 11 of the syringe body. The support 20 of the needle 2 is mounted on the head 13 of the syringe body and the needle-carrier device 3 is screwed onto the support 20 of the needle to fix the needle 2 firmly to the head of the syringe body.

At this point the sleeve 5 is inserted from the rear over the syringe body. During insertion the elastic tongues 31 of the needle-carrier device 3 bend inward, until the teeth 33 of the flexible tongues 31 enter the longitudinal slots 60 in the central part 50 of the sleeve body. At the same time, the longitudinal tongues 56 of the rear part 52 of the sleeve body bend outwards, until the nibs 12 of the collar 11 of the syringe body enter the slots 58 of the tongues 56.

In this situation, as shown in Figures 5 and 6, the teeth 33 of the tongues 31 of the needle carrier device abut against the respective front abutment surfaces 61 of the longitudinal slots 60 of the sleeve and the nibs 12 of the collar of the syringe body are engaged in the respective openings 58 of the longitudinal tongues 56 of the rear part of the sleeve. In this manner, the sleeve 5 is made integral with the syringe body and any axial movement of the sleeve 5 with respect to the syringe body 1 is prevented. It should be noted furthermore that in said situation, the spring 7 is compressed with one end in abutment against the collar 11 of the syringe body and the other end in abutment against the annular abutment surface 55 of the rear part of the sleeve.

Operation of the syringe 100 according to the invention is described below with reference to Figures 7, 8 and 9.

As shown in Figure 7, when the plunger 4 reaches the end of the injection stroke within the chamber of the syringe body, the operating crown 43 of the stem 41 is in contact with the flexible tongues 56 of the rear part of the sleeve. Precisely, the tapered surface 44 of the operating crown of the stem cooperates with the tapered surface 59 of the tongues of the rear part of the sleeve, causing said sleeves 56 to bend outward.

As a result, the nibs 12 of the collar of the syringe body disengage from the openings 58 of the tongues 56. Consequently, axial movement of the sleeve 5 with respect to the syringe body 1 is no longer prevented. Thus through the action of the spring 7 which is released, the sleeve 5 moves axially forward with respect to the syringe body 1 and/or the syringe body 1 moves axially backward with respect to the sleeve 5.

During said axial movement, the sleeve 5 is guided by the teeth 33 of the tongues 31 of the needle-carrier device which slide within the longitudinal slots 60 of the sleeve. When the teeth 33 of the needle-carrier device reach the rear part of the respective slots 60, said teeth 33 push the flexible tongues 63 which bend in the respective notches 65.

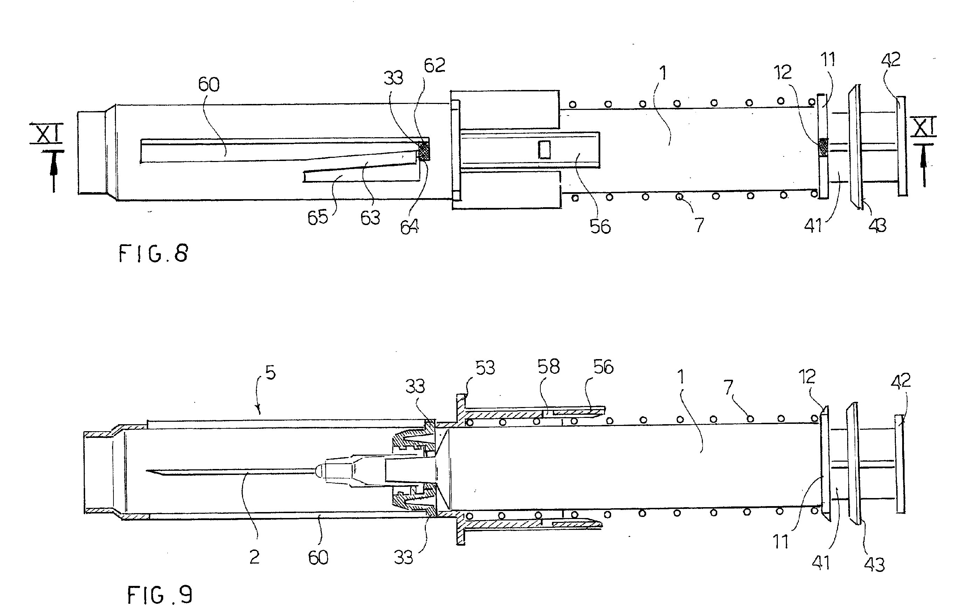

At the end of the stroke of the sleeve 5, as shown in Figures 8 and 9, the teeth 33 of the needle-carrier device abut against the rear abutment surface 62 of the slots 60 of the sleeve. In this situation the needle 2 is protected by the sleeve 5 which is in its forward safety position. It should be noted that in this situation, the flexible tongues 63 of the sleeve return to their initial configuration and thus the teeth of the needle-carrier are blocked between the rear abutment surface 62 of the longitudinal slots of the sleeve and the abutment surface 64 of the flexible tongues 63. In this manner any axial movement of the sleeve with respect to the syringe body is prevented.

In the present description the teeth 33 which slide in the slots 60 of the sleeve have been formed in the needle-carrier 3. However, said teeth 33 can also be formed in the outer surface of the syringe body 1.

Numerous variations and modifications of detail within the reach of a person skilled in the art can be made to the present invention without departing from the scope of the invention, as set forth in the appended claims.

## Claims

1. A disposable syringe (100) comprising:
- a syringe body (1) hollow on the inside and open at the front and rear,
- a plunger (4) sliding inside the syringe body (1) with an injection stroke extending from a retracted syringe filling position to a forward or extended syringe emptying position,, said plunger (4) being provided at the rear with a stem (41) that can be operated manually and extended out of the syringe body through the rear end (42) thereof,
- an injection needle (2) that can be attached to the front end (13) of the syringe body (1) by means of a needle carrier (3),
- a sleeve (5) slidably mounted over said syringe body (1), to pass from a retracted position of use of the syringe to a forward safety position, wherein it covers said needle (2),
- spring means (7) disposed under compression between said sleeve (5) and said syringe body (1) to urge the axial movement of the sleeve (5) with respect to the syringe body,
- locking means (56, 58, 12) provided in the rear part of the sleeve (5) and in the rear part of the syringe body (1), in reciprocal engagement, to keep the sleeve locked in the retracted position of use against the action of the spring means,
- operating means (44) disposed in said stem (41) to release said locking means, when the plunger (4) reaches the end of the injection stroke, so as to allow axial movement of the sleeve into the safety position, thanks to the action of the spring means,
**characterised in that** said sleeve (5) comprises guide means (60) cooperating with complementary guide means (33) integral with the syringe (1) to guide the axial movement of the sleeve (5) with respect to the syringe body.

2. A syringe according to claim 1, **characterised in that** said guide means (5) of the sleeve comprise a pair of longitudinal through slots (60) disposed in diametrically opposite positions in the central part of said sleeve and said guide means integral with the syringe body (1) comprise a pair of teeth or nibs (33), disposed in diametrically opposite positions on the needle-carrier (3) or on the syringe body (1), able to engage in a sliding coupling relationship within the respective longitudinal slots (60).

3. A syringe according to claim 2, **characterised in that** said teeth (33) are disposed on a pair of flexible tongues (31) disposed in diametrically opposite positions on said needle-carrier to be able to bend inwards and said needle-carrier (3) has an inside thread (36) able to engage with an outside thread (22) of a support (20) of the needle that can be fitted to the head (2) of said syringe body.

4. A syringe according to claim 2 or 3, **characterised in that** said longitudinal slot (60) of the sleeve comprises a front abutment and end-of-stroke surface (61) and a rear abutment and end-of-stroke surface (62).

5. A syringe according to claim 4, **characterised in that** within said slot there is provided a longitudinal flexible tongue (63) obtained by means of a notch (65) in the body of the sleeve, parallel to the longitudinal slot (60), so that the flexible tongue (63) creates a narrowing of said longitudinal slot (60) and has an abutment surface (64) opposing said rear abutment and end-of-stroke surface (62) of the longitudinal slot (60).

6. A syringe according to any one of the preceding claims, **characterised in that** said locking means comprise a pair of tongues or nibs (12) protruding radially outward, in diametrically opposite positions, in the rear part of the syringe body and a pair of flexible longitudinal tongues (56) formed in diametrically opposite positions in the rear part (52) of said sleeve (5), said flexible tongues (56) having respective apertures (58) able to receive said respective nibs (12) in a locking relationship.

7. A syringe according to claim 6, **characterised in that** said flexible tongues (56) have a rear part protruding rearward with respect to the rear edge of the sleeve and said operating means of the stem comprise a circular crown (43) disposed in the rear part of the stem (41) cooperating with said rear part of the flexible tongues (56) of the sleeve so as to cause outward bending of the said flexible tongues (56) and disengagement of said nibs (12) from the apertures (58) of the flexible tongues.

8. A syringe according to claim 7, **characterised in that** the rear end of said flexible tongues (56) has a tapered inner surface (59) able to cooperate with a complementary tapered surface (44) provided in the front part of said circular crown (43).

9. A syringe according to any one of the preceding claims, **characterised in that** said spring means comprise a spiral spring (7) disposed around the syringe body (1) with one end abutting against a collar (11) protruding outward from the rear part of the syringe body and the other end abutting against an annular abutment surface (55) provided within the rear part (52) of said sleeve.
